Europäisches Patentamt

(19) ⑲ European Patent Office

Office européen des brevets

(11) Publication number: **0 189 161 B1**

(12) **EUROPEAN PATENT SPECIFICATION**

(45) Date of publication of patent specification: 24.07.91     (51) Int. Cl.⁵: **A61K 37/18, A23L 1/305**

(21) Application number: 86100680.7

(22) Date of filing: 20.01.86

(54) **Enteral nutritional hypoallergenic formula.**

(30) Priority: 29.01.85 US 695993

(43) Date of publication of application:
30.07.86 Bulletin 86/31

(45) Publication of the grant of the patent:
24.07.91 Bulletin 91/30

(84) Designated Contracting States:
**AT BE CH DE FR GB IT LI LU NL SE**

(56) References cited:
**US-A- 4 414 238**

**FOOD SCIENCE & TECHNOLOGY, no.
72-02-A0118, (72005860); J. O'DELL:
"Starches as emulsifiers and stablizers"**

**FOOD SCIENCE & TECHNOLOGY, no.
81-07-T0369 (8104028); M. BUTTOLPH et al.:
"Subchronic studies in cats fed octenyl suc-
cinate - modified food starch " & FOOD &
COSMETICS TOXICOLOGY, vol. 18, no. 4,
1980, pages 357 - 362**

**J. O'DELL: IFST-PROCEEDINGS, vol. 4, no. 2,
1971, pages 77-80**

(73) Proprietor: **ABBOTT LABORATORIES
14th Street and Sheridan Road North St
North Chicago, Illinois 60064(US)**

(72) Inventor: **Mahmoud, Mohamed T.
2061 Cardington Avenue
Columbus Ohio(US)**

(74) Representative: **Modiano, Guido et al
MODIANO, JOSIF, PISANTY & STAUB
Modiano & Associati Via Meravigli, 16
I-20123 Milano(IT)**

# EP 0 189 161 B1

## Description

The invention relates to improved enteral nutritional hypoallergenic formulas and more particularly to hypoallergenic formulas which contain a unique fat emulsifying system.

### Background of the Invention

Hypoallergenic formulas or compositions, which are also referred to as elemental formulas, are characterized in that they contain protein hydrolysates such as soy protein hydrolysate, casein hydrolysate, whey protein hydrolysate or a combination of animal and vegetable protein hydrolysates as a major source of nitrogen. The protein hydrolysates comprise short peptide fragments and/or free amino acids instead of the intact protein found, for example, in cow's milk and soy protein isolate-based formulas. These short peptide fragments and free amino acids have been found to be less immunogenic or allergenic than intact proteins.

In addition to protein hydrolysates, most nutritionally balanced hypoallergenic formulas contain carbohydrates, lipids, vitamins, minerals and are supplemented with additional amino acids to upgrade the nutritional quality of such formulas. These hypoallergenic formulas are utilized for feeding infants, children and adults who have allergies or sensitivities to intact protein, and are often medically used in the treatment of cystic fibrosis, chronic diarrhea, galactosemia, small bowel resection, steatorrhae and protein-calorie malnutrition.

One well known problem in the preparation of hypoallergenic formulas is that extensive protein hydrolysis by acids or enzymes is necessary to provide the short peptides and amino acids utilized in the formulas to render such formulas hypoimmunogenic. These extensively digested and hypoimmunogenic protein hydrolysates have the undesirable characteristic of loss of capacity to emulsify fat and form physically stable emulsions that do not separate during storage.

Another common problem encountered in the preparation of hypoallergenic formulas is the formation of undesirable brown color as a result of the reaction between the carbonyl groups of reducing sugars and the nitrogen-amine-containing compounds such as amino acids in the formula (Maillard type reaction), especially at elevated temperatures encountered during sterilization. In addition to the brown color formation, Maillard type reaction also results in loss of the nutritional value of the protein hydrolysate.

U.S. Patent No. 4,414,238 shows an elemental diet composition comprising carbohydrates, amino acids and/or low molecular weight peptides and lipids. The elemental diet of that patent has a lipid component in the form of an emulsion consisting of lipids, an emulifier selected from the group consisting of mono- and diglycerides, and a corn starch modified with succinic anhydride and is stable and non-browning at a low pH of about 3 to 4.4.

The present invention provides an improved hypoallergenic formula which does not require emulsifiers such as mono- or diglycerides, does not form a brown color at a higher pH range than previous hypoallergenic formulas, and is a stable emulsion. Furthermore, the hypoallergenic formula of the present invention enables suspension of the insoluble calcium and phosphorus salts which are commonly contained in such formulas.

### Summary of the Invention

The present invention is an improved hypoallergenic formula comprising carbohydrates, lipids, a protein hydrolysate, vitamins and minerals and a starch modified by octenyl succinic anhydride which is the sole emulsifying agent. The invention is based on the discovery that the lipophilic moiety of the modified starch of the present invention results in a stable emulsion which is non-browning at a higher pH than previous hypoallergenic formulas.

### Detailed Description of the Invention

The hypoallergenic formula of the invention is made by blending carbohydrates, lipids, and a protein hydrolysate, homogenizing the mixture into a stable emulsion and sterilizing the product in the pH range from 6 to 7.

The protein hydrolysate of the invention may be any suitable protein hydrolysate utilized in a nutritional formula such as soy protein hydrolysate, casein hydrolysate, whey protein hydrolysate, animal and vegetable protein hydrolysates, and mixtures thereof. The protein hydrolysate of the hypoallergenic formula of the invention is preferably a soy protein hydrolysate or a casein hydrolysate comprising short peptides and amino acids. The immunogenicity of the formula of the present invention depends largely on the extent of hydrolysis of the selected protein hydrolysate. To insure hypoimmunogenicity of the formula, the protein hydrolysate should be extensively hydrolyzed to yield very short peptides and free amino acids. This is important since free amino acids and di- and tripeptides are known to be absorbed through the small intestine without any digestive breakdown. Large molecular weight peptides are preferably avoided because they generate a more antigenic formula and cause precipitation and emulsion destabilization. In a preferred embodiment, the protein hydrolysate of the invention contains a high percentage of low molecular weight peptide fragments.

The hydrolyzed protein source of the hypoallergenic formula is also preferably supplemented with various free amino acids to provide a nutritionally balanced amino acid content. Examples of such free amino acids include L-tryptophan, L-methionine, L-cystine, L-tyrosine, L-arginine, taurine and carnitine. The amount of protein hydrolysate and supplemented free amino acid mixture in the hypoallergenic formula may range from 8% to 20% of the total calories of the formula and is preferably in the range of 10% to 14% of total calories.

Carbohydrate sources which may be utilized in the hypoallergenic formula include sucrose and low D.E. maltodextrins and hydrolyzed starches in combination with an octenyl succinic anhydride modified starch. D.E. refers to dextrose equivalent which is a measure of the total reducing power of a carbohydrate source expressed as anhydrous dextrose and calculated as a percent of the total solids. In a hypoallergenic formula, which contains an extensively digested protein hydrolysate, the use of a low D.E. hydrolyzed starch is particularly important to minimize Maillard type reaction and brown color formation. Examples of low D.E. hydrolyzed starches include corn maltodextrin, tapioca maltodextrin and rice maltrodextrin with a D.E. of 10 or lower. Preferably, the carbohydrate source of the hypoallergenic formula is sucrose and an octenyl succinic anhydride modified starch. Examples of octenyl succinic anhydride modified starches which can be utilized in the hypoallergenic formula include corn, tapioca and rice starches modified with octenyl succinic anhydride. The preferred octenyl succinic anhydride modified starch of the invention is octenyl succinic anhydried modified corn starch.

The amount of the total carbohydrate in the hypoallergenic formula may range from 35% to 60% of the total calories of the formula. Preferably, the carbohydrate content is in the range of from 40% to 45% of total calories.

The lipid source of the hypoallergenic formula preferably comprises a mixture of safflower oil, MCT oil (medium chain triglycerides) and soy oil. Examples of other suitable lipid sources for the formula include corn oil, coconut oil, sunflower oil and olive oil. A preferred lipid or lipid mixture for the hypoallergenic formula should contain at least 10% of the fatty acids as linoleic acid (18:2). The amount of the lipid component in the hypoallergenic formula may range from 35% t6 55% of the total calories of the formula and preferably is in the range of 45% to 50%.

The emulsion system of the hypoallergenic formula is solely based on an octenyl succinic anhydride modified starch. The octenyl group of the modified starch imparts hydrophobicity to the starch molecule and greatly increases the affinity of the starch to fat. The succinate gives a net negative charge to the starch molecule which increases the hydrophilic nature of the molecule. Thus, the chemical modification of the starch with octenyl succinic anhydride renders the starch molecule an anionic polyelectrolyte surface active macromolecule. These surface active substances are more effective emulsifiers than the non-ionic surfactants of the mono- and diglycerides. Being a polyelectrolyte surface active macro-molecule, the octenyl succinic anhydride modified starch stabilizes the emulsion by charge stabilization and adsorption at the surface of the fat globule, thus encapsulating the fat globules. The fat globule encapsulation by the starch leads to the formation of a stable emulsion.

Mono- and diglycerides, lecithin and polyglycerol esters of fatty acids were found to be ineffective in producing stable hypoallergenic formula. Hypoallergenic formula made with these emulsifiers yielded unstable emulsions and developed an objectionable cream layer within 24 hours of sterilization. On the other hand, hypoallergenic formula made with octenyl succinic anhydride modified starch exhibited an excellent emulsion stability with negligible cream layer formation after 9 months of storage at room temperature.

The formula also contains a stabilizer such as lambda carrageenan. Lambda carrageenan increases the viscosity of the formula without forming a gel structure, thus retarding the fallout of the insoluble calcium and phosphorus salts used in the formula. Xanthan gum may also be used in hypoallergenic formula as a stabilizer in the same fashion as lambda carrageenan. In addition, the hypoallergenic formula contains vitamins and minerals.

The hypoallergenic formula is formulated with a pH between 6 and 7 to closely simulate the pH of human milk. Low pH values such as 3-4 may cause acidosis in infants fed the hypoallergenic formula.

The ingredients of the hypoallergenic formula of this disclosure are set forth in Table I.

Table I

| Ingredients | Per Liter (676.3 K calories) Preferred Amount | |
|---|---|---|
| Water | 899.0 | g |
| Sucrose | 44.5 | g |
| Modified Corn Starch (octenyl succinic anhydride modified waxy corn starch) (5% $H_2O$) | 26.3 | g |
| Soy Protein Hydrolysate or Casein Hydrolysate | 23.25 | g |
| Safflower Oil | 15.00 | g |
| MCT Oil (medium chain triglycerides) | 18.73 | g |
| Soy oil | 3.75 | g |
| Calcium Citrate | 1.34 | g |
| Calcium Hydroxide | 0.60 | g |
| Calcium Phosphate Monobasic | 0.96 | g |
| Potassium Chloride | 0.22 | g |
| Magnesium Chloride | 0.22 | g |
| Sodium Chloride | 0.13 | g |
| Potassium Citrate | 0.66 | g |
| Ferrous Sulfate | 59.70 | mg |
| Zinc Sulfate | 26.38 | mg |
| Copper Sulfate | 2.69 | mg |
| Manganese Sulfate | 0.37 | mg |
| Carrageenan (lambda type) | 0.52 | g |
| Ascorbic Acid | 0.18 | g |
| Choline Chloride | 70.30 | mg |
| Inositol | 44.82 | mg |
| Alpha-Tocopheryl Acetate | 23.00 | mg |
| Niacinamide | 12.25 | mg |
| Calcium Pantothenate | 7.50 | mg |
| Vitamin A Palmitate | 2.09 | mg |
| Thiamine Chloride Hydrochloride | 0.75 | mg |
| Riboflavin | 0.90 | mg |
| Pyridoxine Hydrochloride | 0.70 | mg |
| Folic Acid | 0.23 | mg |
| Phylloquinone | 0.11 | mg |
| Biotin | 0.05 | mg |
| Vitamin $D_3$ | 0.011 | mg |
| Cyanocobalamin | 4.50 | g /μ |
| L-Cystine dihydrochloride | 0.17 | g |
| L-Methionine | 0.19 | g |
| L-Tryptophan | 0.23 | g |
| L-Tyrosine | 0.11 | g |
| Taurine | 45.0 | mg |
| L-Carnitine | 14.2 | mg |

The following example illustrates a processing procedure for the hypoallergenic formula of the invention.

EXAMPLE

Weighing Procedure

All dry ingredients should be weighed out not more than 12 hours prior to processing of the hypoallergenic formula.

I. Preparation of Oil Blend

A. Weigh out and combine safflower oil, MCT oil and soy oil into a stainless steel steam-jacketed kettle. Heat the oil blend to 60-65°C.

B. Weigh out the fat soluble vitamins consisting of vitamin A palmitate, vitamin D3, alpha-tocopheryl acetate and phylloquinone and add to the oil blend. Maintain the oil blend at the 60-65°C and set aside until used.

II. Mixing Procedure

A. Weigh out proper amount of water and pour into a separate stainless steel steam-jacketed kettle (amount of water is slightly less than formula requires). Reserve a small amount of water to dissolve trace minerals, amino acids and water soluble vitamins. Heat the water to 70-75°C.

B. Weigh out calcium hydroxide, calcium citrate and calcium phosphate monobasic and dissolve in the hot (70-75°C) water.

C. Weigh out and combine the protein hydrolysate, sucrose, octenyl succinic anhydride modified corn starch and carrageenan. Dissolve the combined ingredients in the mineral slurry while stirring constantly.

D. Add the oil blend to the protein hydrolysate/carbohydrate/mineral slurry. Maintain the temperature of the liquid slurry at 70-75°C and hold for 20 minutes while stirring constantly. Adjust the pH of the slurry to 6.0-7.0.

III. Homogenization Procedure

Prior to homogenization, pass the slurry through a preheater to heat the liquid at 79°C. Homogenize one time using double stage homogenizer at a total pressure of 3000-4000 psi ($2.1 \times 10^7$-$2.8 \times 10^7$ Pa) with 500 psi ($3.4 \times 10^6$ Pa) for the second stage. Immediately following homogenization, pass the liquid through a cooler at 4°C. Determine the % solids.

IV. Weigh out the remainder of the minerals and trace minerals and dissolve in water in the following order: sodium chloride, magnesium chloride, potassium chloride, potassium citrate, ferrous sulfate, zinc sulfate, cupric sulfate and manganese sulfate. Add the dissolved mineral solution to the product slurry.

V. Weigh out and dissolve the following amino acids: L-tryptophan, L-methionine, L-cystine-2 HCL, L-tyrosine, taurine and L-carnitine in water and add the dissolved amino acid solution to the product slurry.

VI. A. Weigh out and dissolve the following water soluble vitamins in water: vitamin B1, vitamin B2, vitamin B6, vitamin B12, niacinamide, biotin, folic acid, calcium pantothenate, inositol and choline chloride. Add the dissolved water soluble vitamins to the product slurry.

B. Adjust the pH of the liquid product to 6.0-7.0. Add water to bring the solids to about 13%.

VII. Filling and Sterilization

A. Fill 32 fluid ounce (946 ml) cans (976 gram) or 8 fluid ounce (237 ml) glass bottle or can (244 gram) with the liquid formula.

B. Seam the containers with 16-20 inches Hg ($5.4 \times 10^4$-$6.8 \times 10^4$ Pa) of vacuum.

C. Sterilize the product with an adequate sterilization process as recommended by the FDA for low acid foods.

The advantages of the present invention are 1) a shelf stable ready-to-feed liquid product (with a 12-month shelf life) that has a pH common to human milk which enhances patient acceptability, and 2) a product without browning. Browning is undesirable both because of the appearance and because many of the products of the Maillard reaction are indigestible and cannot be absorbed in the small intestine. These browning reaction by-products are fermented in the colon resulting in acid stool, gas, diarrhea, water and electrolyte losses.

While the hypoallergenic formula is preferably provided in a ready-to-feed liquid form, it may also be concentrated by increasing the percent total solids of the formula or made in powder form, both procedures being well known to those skilled in the art. The concentrate or powder are then reconstituted for feeding by adding water.

## Claims

1. An enteral nutritional hypoallergenic formula comprising carbohydrates, lipids, a protein hydrolysate and a modified starch as an emulsifying agent, characterized in that said starch is a starch modified by octenyl succinic anhydride.

2. The hypoallergenic formula of Claim 1 wherein the formula comprises a stable, non-browning emulsion at a pH from 6 to 7.

3. The hypoallergenic formula of Claim 1 wherein the formula is supplemented with free amino acids.

4. The hypoallergenic formula of Claim 3 wherein the caloric distribution comprises 35% to 60% carbohydrates, 35% to 55% lipids, and 8% to 20% protein hydrolysate and free amino acids.

5. The hypoallergenic formula of Claim 4 wherein the caloric distribution comprises 40% to 45% carbohydrates, 45% to 50% lipids, and 10% to 14% protein hydrolysate and free amino acids.

6. The hypoallergenic formula of Claim 1 further comprising vitamins, minerals and carrageenan.

7. The hypoallergenic formula of Claim 1 wherein the starch comprises octenyl succinic anhydride modified corn starch.

8. The hypoallergenic formula of Claim 1 wherein the carbohydrates comprise sucrose and octenyl succinic anhydride modified corn starch, the lipids comprise safflower oil, medium chain triglycerides, and soy oil, and the protein hydrolysate is selected from the group consisting of soy protein hydrolysate, casein hydrolysate, whey protein hydrolysate, animal and vegetable protein hydrolysates and mixtures thereof.

9. The hypoallergenic formula of Claim 8 wherein the lipids comprise at least 10% linoleic acid.

10. The hypoallergenic formula of Claim 1 wherein the formula is provided as a ready-to-feed liquid, a concentrated liquid or a powder.

**Claims for the following Contracting State: AT**

1. A process for making an enteral nutritional hypoallergenic formula comprising blending a mixture of carbohydrates, lipids, a protein hydrolysate and a modified starch as an emulsifying agent, characterized in that said starch is a starch modified by octenyl succinic anhydride.

2. The process of Claim 1 wherein said mixture is homogenized into a stable emulsion and sterilized at a pH from 6 to 7 wherein said emulsion is non-browning.

3. The process of Claim 1 wherein the formula is supplemented with free amino acids.

4. The process of Claim 3 wherein the hypoallergenic formula has a caloric distribution comprising 35% to 60% carbohydrates, 35% to 55% lipids, and 8% to 20% protein hydrolysate and free amino acids.

5. The process of Claim 4 wherein the hypoallergenic formula has a caloric distribution comprising 40% to 45% carbohydrates, 45% to 50% lipids, and 10% to 14% protein hydrolysate and free amino acids.

6. The process of Claim 1 wherein said mixture further comprises vitamins, minerals and carrageenan.

7. The process of Claim 1 wherein the starch comprises octenyl succinic anhydride modified corn starch.

8. The process of Claim 1 wherein the carbohydrates comprise sucrose and octenyl succinic anhydride modified corn starch, the lipids comprise safflower oil, medium chain triglycerides, and soy oil, and the protein hydrolysate is selected from the group consisting of soy protein hydrolysate, casein hydrolysate, whey protein hydrolysate, animal and vegetable protein hydrolysates and mixtures thereof.

9. The process of Claim 8 wherein the lipids comprise at least 10% linoleic acid.

10. The process of Claim 1 wherein said mixture is blended as part of a ready-to-feed liquid, a concentrated liquid or a powder.

**Revendications**

1. Formule hupoallergénique d'alimentation entérale, comprenant des glucides, des lipides, un hydrolysat de protéines et un amidon modifié comme agent émulsifiant, caractérisée en ce que ledit amidon est un amidon modifié par l'anhydride octényl-succinique.

2. Formule hypoallergénique selon la revendication 1, dans laquelle la formule comprend une émulsion non brunissante, stable, à un pH compris entre 6 et 7.

3. Formule hypoallergénique selon la revendication 1, dans laquelle la formule est additionnée d'aminoacides libres.

4. Formule hypoallergénique selon la revendication 3, dans laquelle la distribution calorique comprend 35 % à 60 % de glucides, 35 % à 55 % de lipides et 8 % à 20 % d'hydrolysat de protéines et d'aminoacides libres.

5. Formule hypoallergénique selon la revendication 4, dans laquelle la distribution calorique comprend 40 % à 45 % de glucides, 45 % à 50 % de lipides et 10 % à 14 % d'hydrolysat de protéines et d'aminoacides libres.

6. Formule hypoallergénique selon la revendication 1, comprenant en outre des vitamines, des minéraux et de la carragénine.

7. Formule hypoallergénique selon la revendication 1, dans laquelle l'amidon comprend de l'amidon de maïs modifié par l'anhydride octényl-succinique.

8. Formule hypoallergénique selon la revendication 1, dans laquelle les glucides comprennent du saccharose et de l'amidon de maïs modifié par l'anhydride octényl-succinique, les lipides comprennent de l'huile de carthame, des triglycérides à chaîne moyenne et de l'huile de soja et l'hydrolysat de protéines est sélectionné parmi le groupe constitué par l'hydrolysat de protéines de soja, l'hydrolysat de caséine, l'hydrolysat de protéines de lactosérum, les hydrolysats de protéines animales et végétales et des mélanges de ces hydrolysats.

9. Formule hypoallergénique selon la revendication 8, dans laquelle les lipides comprennent au moins 10 % d'acide linoléique.

10. Formule hypoallergénique selon la revendication 1, dans laquelle la formule est fournie sous forme de liquide prêt à l'emploi, de liquide concentré ou de poudre.

**Revendications pour l'Etat contractant suivant: AT**

1. Procédé de préparation d'une formule hypoallergénique d'alimentation entérale, comprenant la réalisation d'un mélange de glucides, de lipides, d'un hydrolysat de protéines et d'un amidon modifié comme agent émulsifiant, caractérisé en ce que ledit amidon est un amidon modifié par l'anhydride octényl-succinique.

2. Procédé selon la revendication 1, dans lequel ledit mélange est homogénéisé en une émulsion stable et stérilisé à un pH compris entre 6 et 7 et dans lequel ladite émulsion ne brunit pas.

3. Procédé selon la revendication 1, dans lequel la formule est additionnée d'aminoacides libres.

4. Procédé selon la revendication 3, dans lequel la formule hypoallergénique a une distribution calorique comprenant 35 % à 60 % de glucides, 35 % à 55 % de lipides et 8 % à 20 % d'hydrolysat de protéines et d'aminoacides libres.

5. Procédé selon la revendication 4, dans lequel la formule hypoallergénique a une distribution calorique comprenant 40 % à 45 % de glucides, 45 % à 50 % de lipides et 10 % à 14 % d'hydrolysat de protéines et d'aminoacides libres.

6. Procédé selon la revendication 1, dans lequel ledit mélange comprend en outre des vitamines, des minéraux et de la carragénine.

7. Procédé selon la revendication 1, dans lequel l'amidon comprend de l'amidon de maïs modifié par l'anhydride octényl-succinique.

8. Procédé selon la revendication 1, dans lequel les glucides comprennent du saccharose et de l'amidon de maïs modifié par l'anhydride octényl-succinique, les lipides comprennent de l'huile de carthame, des triglycérides à chaîne moyenne et de l'huile de soja et l'hydrolysat de protéines est sélectionné parmi le groupe constitué par l'hydrolysat de protéines de soja, l'hydrolysat de caséine, l'hydrolysat de protéines de lactosérum, les hydrolysats de protéines animales et végétales et des mélanges de ces hydrolysats.

9. Procédé selon la revendication 8, dans lequel les lipides comprennent au moins 10 % d'acide linoléique.

10. Procédé selon la revendication 1, dans lequel ledit mélange est mélangé en tant que partie d'un liquide prêt à l'emploi, d'un liquide concentré ou d'une poudre.

## Patentansprüche

1. Enterale, hypoallergene Ernährungszusammensetzung, umfassend Kohlenhydrate, Lipide, ein Proteinhydrolysat und eine modifizierte Stärke als Emulgiermittel, dadurch gekennzeichnet, daß diese Stärke eine durch Octenylbernsteinsäureanhydrid modifizierte Stärke ist.

2. Hypoallergene Zusammensetzung nach Anspruch 1, worin die Zusammensetzung eine stabile, nicht braunwerdende Emulsion bei einem pH-Wert von 6 bis 7 darstellt.

3. Hypoallergene Formulierung nach Anspruch 1, worin die Zusammensetzung durch freie Aminosäuren ergänzt ist.

4. Hypoallergene Zusammensetzung nach Anspruch 3, worin die Brennwertverteilung 35 bis 60 % Kohlenhydrate, 35 bis 55 % Lipide und 8 bis 20 % Proteinhydrolysate und freie Aminosäuren umfaßt.

5. Hypoallergene Zusammensetzung nach Anspruch 4, worin die Brennwertverteilung 40 bis 45 % Kohlenhydrate, 45 bis 50 % Lipide und 10 bis 14 % Proteinhydrolysate und freie Aminosäuren umfaßt.

6. Hypoallergene Zusammensetzung nach Anspruch 1, weiterhin umfassend Vitamine, Minerale und Carrageenan.

7. Hypoallergene Zusammensetzung nach Anspruch 1, worin die Stärke mit Octenylbernsteinsäureanhydrid modifizierte Maisstärke umfaßt.

8. Hypoallergene Zusammensetzung nach Anspruch 1, worin die Kohlenhydrate Saccharose und mit Octenylbernsteinsäureanhydrid modifizierte Maisstärke umfassen, die Lipide Safloröl, mittelkettige Triglyzeride und Sojaöl umfassen, und das Proteinhydrolysat aus der aus Sojaproteinhydrolysat, Kaseinhydrolysat, Molkeproteinhydrolysat, Hydrolysaten aus tierischem und pflanzlichem Protein und Gemische hievon umfassenden Gruppe ausgewählt ist.

9. Hypoallergene Zusammensetzung nach Anspruch 8, worin die Lipide wenigstens 10 % Linolsäure umfassen.

10. Hypoallergene Zusammensetzung nach Anspruch 1, worin die Zusammensetzung als verabriechungsfertige Flüssigkeit, konzentrierte Flüssigkeit oder als Pulver ausgebildet ist.

## Patentansprüche für folgenden Vertragsstaat: AT

1. Verfahren zur Herstellung einer enteralen, hypoallergenen Ernährungszusammensetzung,umfassend das Vermischen eines Gemisches aus Kohlenhydraten, Lipiden, einem Proteinhydrolysat und einer modifizierten Stärke als Emulgiermittel, dadurch gekennzeichnet, daß diese Stärke eine durch Octenylbernsteinsäureanhydrid modifizierte Stärke ist.

2. Verfahren nach Anspruch 1, worin das Gemisch zu einer stabilen Emulsion homogenisiert und bei einem pH-Wert von 6 bis 7 sterilisiert wird, wobei diese Emulsion nicht braunwerdend ist.

3. Verfahren nach Anspruch 1, worin die Zusammensetzung durch freie Aminosäuren ergänzt wird.

4. Verfahren nach Anspruch 3, worin die hypoallergene Zusammensetzung eine Brennwertverteilung aufweist, die 35 bis 60 % Kohlenhydrate, 35 bis 55 % Lipide und 8 bis 20 % Proteinhydrolysate und freie Aminosäuren umfaßt.

5. Verfahren nach Anspruch 4, worin die hypoallergene Zusammensetzung eine Brennwertverteilung aufweist, die 40 bis 45 % Kohlenhydrate, 45 bis 50 % Lipide und 10 bis 14 % Proteinhydrolysate freie Aminosäuren umfaßt.

6. Verfahren nach Anspruch 1, worin das Gemisch zusätzlich Vitamine, Minerale und Carageenan umfaßt.

7. Verfahren nach Anspruch 1, worin die Stärke mit Octenylbernsteinsäureanhydrid modifizierte Maisstärke umfaßt.

8. Verfahren nach Anspruch 1, worin die Kohlenhydrate Saccharose und mit Octenylbernsteinsäureanhydrid modifizierte Maisstärke umfassen, die Lipide Safloröl, mittelkettige Triglyzeride und Sojaöl umfassen und das Proteinhydrolysat aus der aus Sojaproteinhydrolysat, Kaseinhydrolysat, Molkeproteinhydrolysat, Hydrolysaten von tierischem und pflanzlichem Protein deren Gemische umfassenden Gruppe ausgewählt ist.

9. Verfahren nach Anspruch 8, worin die Lipide wenigstens 10 % Linolsäure umfassen.

10. Verfahren nach Anspruch 1, worin das Gemisch als Teil einer verabreichungsfertigen Flüssigkeit, einer konzentrierten Flüssigkeit oder eines Pulvers eingemischt wird.